(19) Europäisches Patentamt / European Patent Office / Office européen des brevets

(11) EP 4 631 513 A1

(12) EUROPEAN PATENT APPLICATION
published in accordance with Art. 153(4) EPC

(43) Date of publication:
15.10.2025 Bulletin 2025/42

(21) Application number: 22967994.9

(22) Date of filing: 13.12.2022

(51) International Patent Classification (IPC):
A61K 36/28 $^{(2006.01)}$    A61K 31/728 $^{(2006.01)}$
A61P 27/02 $^{(2006.01)}$

(52) Cooperative Patent Classification (CPC):
A61K 31/728; A61K 36/28; A61P 27/02

(86) International application number:
PCT/MX2022/050122

(87) International publication number:
WO 2024/123165 (13.06.2024 Gazette 2024/24)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 09.12.2022 MX 2022015859

(71) Applicant: Centro de Retina Médica Y Quirúrgica,
S.C.
Zapopan, 45116 (MX)

(72) Inventors:
• SANTOS GARCÍA, Arturo
Zapopan, 45116 (MX)
• ALTAMIRANO VALLEJO, Juan Carlos
Zapopan, 45116 (MX)
• MORFIN HERAS, Pedro
Zapopan, 45116 (MX)

(74) Representative: Herrero & Asociados, S.L.
Cedaceros, 1
28014 Madrid (ES)

(54) **OPHTHALMIC FORMULATION FOR TOPICAL USE CONTAINING HELENALIN, 11A,13-DIHYDRO HELENALIN AND FLAVONOIDS FROM ARNICA MONTANA EXTRACT AND SODIUM HYALURONATE, AND USE THEREOF BY HUMANS TO TREAT DRY EYE SYNDROME**

(57) The present invention relates to the field of biotechnology and medicine. The invention consists of a formulation for topical ophthalmic use that contains Helenalin, 11-$\alpha$, 13-dihydrohelenalin and flavonoids from Arnica Montana extract in combination with sodium hyaluronate for the primary treatment and/or as an adjuvant of dry eye syndrome in humans.

**Dry eye syndrome** is one of the most commonly observed clinical conditions worldwide with an estimated population occurrence of 9 - 30%. In addition, Dry Eye Syndrome has very pertinent inflammatory and oxidative aspects which require diverse strategies to help in its treatment. The availability of a formulation which has anti-inflammatory and antioxidant properties in addition to a lubricating function is of great interest to the scientific and medical community.

Fig. 3

EP 4 631 513 A1

**Description**

**FIELD OF INVENTION**

The present invention relates to the field of biotechnology and medicine. The invention consists of a formulation for topical ophthalmic use that contains Helenalin, 11-$\alpha$, 13-dihydrohelenalin and flavonoids from Arnica Montana extract in combination with sodium hyaluronate for the primary treatment and/or as an adjuvant of dry eye syndrome in humans.

**BACKGROUND OF THE INVENTION**

**Arnica Montana** is a plant which has been widely used in herbalism and homeopathy for decades. Also known *as wolf bone, mountain tobacco or leopard energy,* it has been used in the treatment of various diseases such as rheumatoid arthritis, systemic lupus erythematosus, hematomas and inflammatory conditions in general. Originally from central and southern Europe, the plant spread rapidly throughout Asia and North America and in all cases has been used as the main constituent of multiple remedies. It is a plant with an upright stem that reaches 15 to 60 cm in height and its oval leaves form a basilar rosette on the ground. The yellow flowers are large and terminal, the stem is about 30 cm high, hollow, hairy and rough, branches placed 2 by 2, simple, straight, naked and with a terminal yellow flower. The leaves are oval and similar to those of the plantain, rough on top and hairless on the underside.

In many Western countries (including Mexico), the word "arnica" is widely associated with the remedy for trauma sequelae, with topical dermatological preparations being preferred by the general population. To date, there are no public reports or studies reported that demonstrate adverse effects in its topical use, and homeopathic and topical formulas are recommended by surgeons to assist in the treatment of patients to relieve postoperative ecchymoses, edema, hematomas and/or pain. The positive effects are associated with the fact that Arnica Montana contains phenolic components such as helenalin, 11-$\alpha$, 13-dihydrohelenalin and various flavonoids. However, there is no ophthalmic product for topical use, which is the basis for this invention. This medicinal plant contains flavonoids, the most important for the functions they perform are: quercetin, *isoquercetin, rutin, kaempherol-3-O-glucoside and apigenin-7-O-glucoside.* It also contains phenolic acids such as helenalin, 11-$\alpha$, 13-dihydrohelenalin, bitter substances and amacin, among other components, which give this medicinal herb anti-inflammatory, antioxidant, analgesic, rubefacient, and astringent properties.

**Dry Eye Syndrome (DES)** is one of the most common clinically observed conditions worldwide, with an estimated prevalence of 9-30% of the population. However, several scientific reports show that the widespread use of computers and monitors, especially since the SARS CoV-2 pandemic, has increased the impact of eye strain related to visual function related to dry eye in up to 70.8% and 18.3% of people who use protective masks (mouth covers), causing dry eye associated with masks.

Today, artificial tear instillation has become the preferred therapeutic approach (gold standard) for DES. However, compliance and adherence to treatment is as low as 10.2%, since 6 out of 10 patients use drops only when necessary to relieve the subjective symptoms caused by DES. This causes these patients to seek home remedies in up to 76% of cases, causing frustration because dry eye is perceived as an "*old people's disease*" or as something less than a seasonal allergy. For example, research is being carried out on various therapeutic approaches to avoid low adherence, reduce DES symptoms and increase patient comfort.

In particular, many ocular surface diseases, including DES, are characterized by significant overproduction of ROS (reactive oxygen species), oxidative stress, and underlying inflammatory mechanisms. Hyperosmolarity, a hallmark of DES, increases the expression of proinflammatory cytokines, chemokines, and MMPs (matrix metalloproteinases). Among the MMPs that are produced in excess during states of hyperosmolarity of the ocular surfaces are MMP-2, MMP-3 and significantly MMP-9, the latter being the most relevant MMP in measurement tests in clinical studies (Quidel, Test InflammaDry®). On the other hand, ROS leads to cellular oxidative damage that also contributes to inflammation. Inflammation on the surface of the eyeball associated with DES is usually chronic and progresses to include activation of oxidative stress pathways, increased expression of the cytokines involved, for example, TNF-a, IL-1β, vascular endothelial growth factor (VEGF), intercellular adhesion molecule-1 (ICAM-1), vascular cell adhesion molecule-1 (VCAM-1) and nuclear factor NFkB.

In this vein, there are some studies that are used to assess inflammation on the surface of the eyeball and abnormal changes in its morphology. Impression cytology of the surface of the eyeball is a non-invasive and precise method to evaluate morphological abnormalities of the cornea and conjunctiva. To do this, Millipore filters are used to collect samples and Papanicolaou stains are used to assess the epithelium and/or basal membranes. With this method you can observe the morphology of the epithelium, limbal stem cells, conjunctival cells (goblet cells), presence of inflammatory cells, as well as changes in their morphology and number. The main point is that the more inflammation is present on the surface of the eyeball due to DES, the greater the morphological abnormalities that will be presented and that can be analyzed by this test.

Arnica Montana has significant anti-inflammatory potential. Huber et al. in 2011 revealed that the molecular mechanism of sesquiterpene lactones (SL) differs from nonsteroidal anti-inflammatory drugs (NSAIDs), i.e., indomethacin and acetylsalicylic acid, as these lactones significantly decrease NF kappa-B-mediated inflammation. The phosphorylation and degeneration of Ikappa-B, an inhibitory subunit of NF-kappa-B, stimulates the production of NF-kappa-B. The activation of NF kappa-B by T cells, B cells, and epithelial cells is inhibited by helenalin, and 11-$\alpha$, 13-dihydrohelenalin, which are the most prevalent and relevant SL contained in *Arnica Montana,* which in turn block the gene expression that drives the production of NF kappa-B. This blockade is precise and is due to the alteration of the NF-kappa-B/I kappa-B complex, through the inhibition of the release of Ikappa-B by helenalin and 11-$\alpha$, 13-dihydrohelenalin.

Arnica Montana has been investigated for its anti-inflammatory potential and scientific publications indicate that it significantly reduces inflammation and edema in rats, since it inhibits the action of histamine, increasing vascular permeability. Likewise, this research demonstrated that when a solution of *Arnica Montana,* dexamethasone or 5% hydroalcoholic solution was injected into these rats, anti-inflammatory activity was triggered. Kawakami et al. reported in 2011 a series of positive inflammatory cells, which play an important role in the inflammatory process, such as CD54 (ICAM-1), CD18 (BETA 2 integrin), CD45RA (B lymphocytes), CD3 (T lymphocytes), CD163 (ED2 protein) and MAC 387 (monocytes and macrophages) in rats. It was concluded that rats that presented chronic edema exhibited less edema, less mast cell degranulation and an increase in the diameter of lymphatic vessels after being exposed to *Arnica Montana.* In another study, it was concluded that acute non-fibrous mastitis disease can be effectively treated with *Arnica* when taken orally in combination with Healwell VT-6 (comprising Calcium fluoricum 200 CH, Conium 30 CH, Silicea 30 CH, Phytolacca 200 CH, iBelladonna 30 CH, Ipecacuanha 30 CH and Bryonia 30 CH). It has also been reported by Clair et al., that the use of *Arnica Montana* when administered with herbs such as Rue, Willow Bark, St. John's Wort and Comfrey will have a therapeutic function by increasing musculoskeletal healing in cases of deep pain, such as arthritis or in the first 24-48 hours after trauma. Likewise, Arnica Montana extract shows significant antioxidant activity. The DPPH (2,20-diphenyl-1-picrylhydrazyl radical) free radical scavenging method and the phosphomolybdate method have been used to determine the antioxidant potential of this plant. At a concentration of 5 mg/ml, *Arnica Montana* exhibits a removal potential of 71.52% in the DPPH method and 63.68% of the total antioxidant activity (phosphomolybdate method) which is mainly attributed to the presence of flavonoids and phenolic compounds such as quercetin. Multiple studies have verified the anti-inflammatory activity of quercetin, which inhibits tumor necrosis factor-$\alpha$ (TNF-$\alpha$), interleukins (IL-8, IL-1 $\alpha$), the production of inflammation-producing enzymes (cyclooxygenase and lipoxygenase), the inhibition of cytokines, interferons and MMPs, including MMP-9. Camargo et al. in 2013, evaluated the effect of *Arnica Montana* on mitochondrial oxidative stress induced by $Ca^{2+}$ and inorganic phosphate and/or lipid peroxidation mediated by $Fe^{2+}$ citrate. through alterations in oxygen consumption rates using mitochondrial suspensions prepared from livers of male rats of the Wistar strain. The use of Arnica Montana produced a notable reduction in mitochondrial $O_2$ consumption and provided protection against $Ca^{2+}$ and phosphate induced hepatic mitochondrial membrane permeability by inorganics, $Fe^{2+}$ citrate-mediated lipid peroxidation and ROS-mediated protein fragmentation.

On the other hand, the extraction method of the active elements contained in *Arnica Montana* varies greatly depending on the formulation and the manufacturer. Many of these methods remove active components such as Helenalin, 11-$\alpha$, 13-dihydrohelenalin and flavonoids during the *Arnica Montana* extraction process. In order to maximize the yield of the active elements contained in Arnica Montana and prioritize its anti-inflammatory effects, a topical ophthalmic formulation containing *Arnica Montana* extract should use ethanol extraction, as this allows concentration of helenalin and 11-$\alpha$, 13-dihydrohelenalin to greater than 4%. The above is of great relevance, since, according to the European Pharmacopoeia, all products based on *Arnica Montana* with lower concentrations of helenalin and/or 11-$\alpha$, 13-dihydrohelenalin lack pharmacological activity and are classified as homeopathic medicine products, which provide no clinical evidence of any function in the tear film of people with dry eye syndrome. Finally, the sterilization method used for these formulations is also of great relevance to preserve these active elements and their properties.

Sodium hyaluronate has been used as a topical ocular lubricant compound in multiple commercial products for the treatment of dry eyes such as Dropstar® TG (Farmigea, Italy), VISMED® (Chemedica, Switzerland), Vishibe® (Thea, United States; Chemedica , Switzerland), Hyasol (manufactured by Lab Pablo Cassara SRL for Bausch & Lomb Argentina SRL) Hyalistíl (Sifi, Italy), Lalurex Ipotonico (Fidia, Italy), Hialudorf (PJbairnaDorf, Argentina), Qxyal (Santen, Germany), Lacripharma (ICN Argentina), Dunason® (Alcon Laboratorios De Brasil, Ltd.), and Maxus (Bausch & Lomb Argentina), whose function is widely described in the literature, *but which lacks an anti-inflammatory and antioxidant function.*

Likewise, the use of preservative-free formulations is a favorable trend in products for the treatment of dry eye, since, in some people, dry eye can be exacerbated by the use of preservatives in topical ophthalmic formulations used as a treatment for the same. For that reason, the sterilization method and the different technologies of bottles and/or containers used to preserve and contain said formulations are of great relevance to enable formulations free of preservatives.

As already mentioned, DES (Dry Eye Syndrome) has a very relevant inflammatory and oxidative component and requires very diverse strategies contributing to its treatment. The use of a formulation that has anti-inflammatory and antioxidant properties in combination with lubricating action is of great interest to the scientific community.

Based on the analysis of prior art documents, there are inventions and products that try to solve similar problems, such as the **Arnistil Eye Drops**, which are commercially available in Europe. The formulation that characterizes these drops presents significant differences when compared to the formulation that supports the present invention, among which the presence of glycerin at 0.1% and the NON-THERAPEUTIC concentration of Arnica Montana at 0.1% stand out. Formulations containing Arnica Montana extract, commercially available in Europe (including those for ophthalmic use), are fundamentally based on traditional preparations with very limited clinical proof of efficacy according to the European Medicines Agency, since there are no clinical trials that support them. Likewise, these types of products lack preparations focused on the use of helenalin, 11-$\alpha$, 13-dihydrohelenalin and flavonoids from said extracts, as well as evidence of their use and effectiveness in dry eye syndrome with objective tests such as MMP-9 measurement and/or impression cytology. Based on the above, the design and development of a formulation that contains the appropriate therapeutic concentrations of helenalin, 11$\alpha$, 13-dihydrohelenalin and flavonoids from Arnica Montana extract that supports the tolerability, safety and efficacy profile, in the results obtained through clinical trials with objective tests such as the measurement of MMP-9 and/or cytology impression of patients with active symptoms associated with dry eye syndrome is a pending issue, which the formulation of the present invention resolves for the first time.

The US Patent US10231990, entitled "PRESERVATIVE-FREE COMPOSITION FOR TOPICAL USE INCLUDING HYALURONIC ACID", is a patent granted on March 19, 2019 that describes topical dermatological use of hyaluronic acid as a method to prevent or treat dry eye in patients by administering it to the eyelid. It is very important to clarify that this patent does not describe administration on the surface of the eyeball and only describes its topical application on the eyelid (skin). Additionally, the same patent describes the possible presence of additional bioactive compounds in it, such as vitamin A and sucralfate. However, it does not describe the use of bioactive *Arnica Montana* extract. It is more than clear that the document does not provide evidence of the effect of the formulation protected by the cited patent on the inflammatory molecular process associated with dry eye syndrome and fatigue due to the use of monitors.

The Chinese patent CN107929198 entitled "EYE CREAM FORMULA", dated April 20, 2018, is a patent that describes a cosmetic cream product for the treatment of the skin (eyelids), with a moisturizer effect, which should be applied to the bags under the eyes to improve skin wrinkle lines. This cream is used as a thin protective layer on the skin around the eyes; It is more than evident that the cosmetic cream described above is not designed for ophthalmic use and administration on the ocular surface. The formulation of said patent has a large number of active compounds that are not used in the treatment of dry eye syndrome and fatigue due to the use of monitors; It does not describe any inhibition of the inflammatory process of the ocular surface, dependent on the nuclear transcriptional factor NF-kB, nor on the expression of inflammatory cytokines, nor matrix metalloproteases, which play a significant role in inflammation and symptomatology. associated with dry eye syndrome.

The Mexican Patent MX390524, granted on February 25, 2022, was filed by the same group of inventors as the present invention. The difference between the two inventions is that patent MX390524 exclusively describes the characteristics of the formulation, and the new one has as its main objective its applications for the primary and/or adjuvant treatment of dry eye by improving the quality of the tear film, modulating the inflammatory process and the symptoms associated with it, as well as 'visual fatigue' due to the use of monitors ('Computer vision syndrome' or 'visual strain' in English), so the present invention can be considered an extension of MX390524 in terms of application. The present invention describes the application of the formulation described in the patent title MX390524 to improve the quality of the tear film, modulate the inflammatory process and the symptoms associated with dry eye syndrome with an ophthalmic formulation for topical use that contains Helenalin, 11$\alpha$, 13-dihydrohelenalin and flavonoids from *Arnica Montana* extract in combination with sodium hyaluronate for primary treatment and/or as a coadjuvant for dry eye syndrome in humans. The active compounds helenalin, 11$\alpha$, 13-dihydrohelenalin from *Arnica Montana* extract present in this formulation (*Arnica Montana* extract 100mg/mL) selectively inhibit the expression of proinflammatory cytokines (IL-1, IL-2, IL-6, IL-8 and TNF-$\alpha$) and matrix metalloproteinases (MMPs) associated with the inflammatory process of the ocular surface responsible for the symptoms of dry eye syndrome (DES) and from the use of monitors, by blocking the nuclear transcription factor NF-kB. The universally accepted and available methodology for measuring this effect is the quantification of matrix metalloprotease-9 (MMP-9) (InflammaDry MMP-9 test), from the commercial company Quidel.

For all of the above, the present invention consists of a formulation for topical ophthalmic use that contains Helenalin, 11-$\alpha$, 13-dihydrohelenalin and flavonoids from *Arnica Montana* extract in combination with sodium hyaluronate for the primary treatment and/or as an adjuvant in the treatment of the dry eye syndrome in humans. In order to demonstrate its action on the tear film and its modulation of the inflammatory process and the symptoms associated with dry eye syndrome, a clinical study was carried out in subjects with mild to moderate dry eye syndrome and active symptoms. The technical details of the developed invention are described below.

## OBJECT OF THE INVENTION

The object of this invention is to make available an ophthalmic topical formulation for use in humans that has anti-inflammatory and antioxidant properties in combination with a lubricating action. The present invention consists of a formulation for topical ophthalmic use containing helenalin, 11-$\alpha$, 13-dihydrohelenalin and flavonoids from *Arnica Montana* extract in combination with sodium hyaluronate for the primary treatment and/or as an adjuvant for the treatment of dry eye syndrome in humans.

The invention which is useful to improve the quality of the tear film, modulate the inflammatory process and the symptoms associated with dry eye syndrome, contains:

i. A component with anti-inflammatory and antioxidant properties and a lubricant containing helenalin, 11-$\alpha$, 13-dihydrohelenalin and flavonoids from *Arnica Montana* extract and sodium hyaluronate which comprises, per 1 mL;

ii. Arnica Montana Extract: 100-500 mg;

iii. Sodium hyaluronate: 1 - 4 mg

**BRIEF DESCRIPTION OF THE FIGURES**

Figure 1 represents the design of the tolerability and safety study where the study formulation is the ophthalmic topical formulation containing helenalin, 11-$\alpha$, 13-dihydrohelenalin and flavonoids from *Arnica Montana* extract in combination with sodium hyaluronate.

Figure 2 represents the design of the efficacy study. During the study, all subjects attended a baseline visit and were assigned to 1 of 2 groups. Additionally, all subjects had a minimum follow-up period of 4 weeks (28 days).

Figure 3 represents images of the safety study. The images indicate the same eye of the same patient in his baseline shot (A) with (blue light) and without (white light), in their week 1 (B) and week 3 (C) shot. These images demonstrate that there were no changes in ocular surface staining after using the study formulation.

Figure 4 represents a diagram of the effectiveness study; This diagram shows the subjects enrolled in the efficacy study, admitted, randomized and analyzed in the two groups (group A = study formulation) and group C = control with ocular lubricant (Eyestil lub®).

Figure 5 is a schematic representation of the impression cytology procedure employed by the inventors.

Figure 6 illustrates representative images of impression cytology at baseline (1-A) and month 1 (1-B) in the study group. All patients exhibited statistically significant improvement.

Figure 7 illustrates representative images of the impression cytology at baseline (2-A) and month 1 (2-B) in the control group. None of the patients exhibited improvement and some of them worsened.

**DETAILED DESCRIPTION OF THE INVENTION**

The characteristic details of the ophthalmic formulation for topical use containing helenalin, 11-$\alpha$, 13-dihydrohelenalin and flavonoids from *Arnica Montana* extract in combination with sodium hyaluronate for the primary treatment and/or as an adjuvant in the treatment of dry eye syndrome in humans. Another object is to improve the quality of the tear film, modulate the inflammatory process and the symptoms associated with dry eye syndrome. These positive effects are clearly demonstrated in the following description and in the illustrative images that are attached, serving as a reference to indicate the tests carried out.

The object of this invention is to make available a formulation for topical ophthalmic use for use in humans with applications to improve the quality of the tear film, modulate the inflammatory process and the symptoms associated with dry eye syndrome.

**The formulation comprises:**

a) A component with anti-inflammatory and antioxidant properties and a lubricant containing helenalin, 11-$\alpha$, 13-dihydrohelenalin and flavonoids from *Arnica Montana* extract in combination with sodium hyaluronate for primary treatment and/or as adjuvant in the treatment of dry eye syndrome in humans comprising per 1 mL:

i. Arnica Montana Extract: 100-500 mg
ii. Sodium hyaluronate: 1 - 4 mg

Additionally, it is important to mention that the <u>formulation is protected by patent by the inventors of this new invention</u> and the object of this new invention is its use and applications to improve the quality of the tear film, modulate the inflammatory process and symptoms. associated with dry eye syndrome.

The applications and uses of said formulation include the following applications wherein a series of studies have been carried out that demonstrate the following applications and uses:

- Anti-inflammatory, antioxidant and lubricating properties that ameliorate symptoms associated with dry eye syndrome and fatigue due to the use of monitors (computer vision syndrome or visual strain);

- Moreover, vascular permeability is improved through histamine modulation, with the potential to modify the inflammatory cascade via selective inhibition of transcriptional factors;

- Moreover, vascular permeability is improved through histamine modulation, with the potential to modify the inflammatory cascade via selective inhibition of transcriptional factors.

*Clinical study to verify the applications in dry eye syndrome and fatigue due to the use of monitors - computer vision syndrome - (computer vision syndrome or - visual strain) of a formulation containing helenalin, $11\alpha$, 13-dihydrohelenalin and flavonoids from Arnica Montana extract in combination with sodium hyaluronate.*

In order to verify these applications and uses of the formulation, the favorable, unexpected and not previously described results of a clinical study of the use of the formulation for topical ophthalmic use in humans that have anti-inflammatory and antioxidant properties together with a lubricating action are presented. The present invention consists of a formulation for topical ophthalmic use that contains helenalin, 11-$\alpha$, 13-dihydrohelenalin and flavonoids from *Arnica Montana* extract in combination with sodium hyaluronate for use in the treatment of dry eye syndrome. The invention is useful for improving the quality of the tear film, modulating the inflammatory process and symptoms associated with dry eye syndrome, which includes:

A formulation for use in improving the quality of the tear film, modulating the inflammatory process and symptoms associated with dry eye syndrome in humans on a 1 ml basis comprising:

a) Arnica Montana extract: 100-500 mg,

b) Sodium hyaluronate: 1 - 4 mg,

wherein this formulation additionally has anti-inflammatory, antioxidant and lubricating properties.

To verify these positive effects on the surface of the eyeball, a prospective, randomized, double-blind phase I-II clinical study was carried out, with a control group (placebo) of 2 study groups. Additionally, **in order to verify the extent of the effects of the *Arnica Montana* extract, a dilution of the initial range was made of the 1 mL content, where initially the *Arnica Montana* extract is from 100 to 500 mg, the capacity of the *Arnica Montana* extract was verified in a range of 2 to 100 mg per 1 ml of the formulation.**

The study was carried out in two parts. The first part (tolerability and safety study) was carried out in 20 healthy subjects, in a single prospective and blind study group, where the tolerability and effectiveness of the formulation was evaluated. The second part of the clinical study (efficacy) was carried out on 48 eyes of 48 patients with a diagnosis of mild to moderate dry eye syndrome, with use of monitors > 8 hours a day who met all the inclusion and exclusion criteria of the study (OSDI > 13, Tear breakup time <10 seconds and MMP-9 positivity). The clinical study was carried out in a single center, in patients with a diagnosis of mild and moderate dry eye syndrome with use of monitors >8 hours a day, in a private research unit in ophthalmology with ISO 9001:20015 certification in 2021. The patients were divided into 2 different groups:

- Group using the formulation reason for this invention as primary treatment (Study Group = A).

- Group using ocular lubricant (0.4% sodium hyaluronate commercially available as primary treatment (Eyestil lub® Laboratorios SI FI, Mexico), (Control Group = C).

Approval clearance was obtained from the internal review board prior to patient enrollment (Ethics and Research Committee and Research Committee). This study adhered to the principles of the Declaration of Helsinki.

The protocol also adhered to the International Conference on Harmonization (ICH) of Good Clinical Practice guidelines and all other applicable local regulatory requirements and laws prior to enrollment, written informed consent was obtained from all subjects after a full explanation of the nature of the study and possible adverse events (AEs) of the study.

Subjects over 18 years of age, with a diagnosis of mild to moderate dry eye syndrome according to OSDI (ocular surface disease index), non-invasive tear film breakup time (NIF-BUT) <10 seconds and any degree of corneal staining with sodium fluorescein and conjunctival staining with lissamine green, with positive measurement for MMP-9 using the Quidel InflammaDry® kit, who suffered visual stress and screen work >8 hours/day.

Intervention:

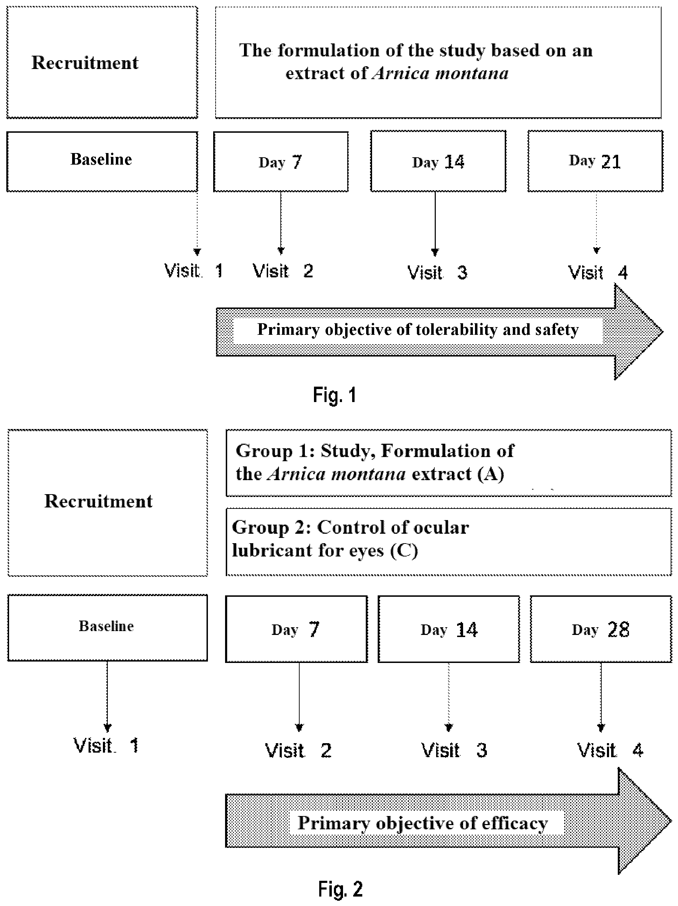

1. Tolerability and safety study. The 20 subjects were included in a single study group (Study formulation based on *Arnica Montana* extract and sodium hyaluronate) patients were instructed to administer one drop 3 times a day. The intervention period was 3 weeks (21 days) and a tolerability questionnaire (ocular tolerability) was completed by all patients. Figure 1 demonstrates the design of the tolerability and safety study; In this case, the AcuSense drops® product is the same as the formulation for topical ophthalmic use that contains Helenalin, 11-$\alpha$, 13-dihydrohelenalin and flavonoids from Arnica Montana extract in combination with sodium hyaluronate.

**2. Efficacy study**. Subjects were randomly assigned to one of two study groups that included the use as the primary treatment of the study formulation (A) (formulation described in this document), or the ocular lubricant use group (Eyestil lub®, Laboratories SI FI, Mexico) (C). Patients in both groups were instructed to apply one drop of the corresponding formulation three times a day for 4 weeks (28 days). During each of the visits, the subjects were subjected to different studies:

- Evaluation of symptoms using the OSDI (Ocular Surface Disease Index) questionnaire. This questionnaire is the most used by ophthalmologists to evaluate dry eye symptoms. If the score is > 13, it is considered a positive criterion for the diagnosis of dry eye;

- Measurement of best corrected visual acuity (BCVA). The best-corrected visual acuity measurement was performed based on the ETDRS protocol, which is the most widely used vision measurement protocol in clinical research;

- Complete clinical ophthalmological examination using a slit lamp. This test is non-invasive and is the test most used by ophthalmologists to assess the general condition of the eyeball;

- Objective measurement of non-invasive tear breakup time (NIF-BUT) and average time (NIAvg-BUT) using the Schwind Sirius® topography equipment (CSO SRL, Italy);

- MMP-9 sampling with the Quidel equipment (InflammaDry®). This is the most specific test to objectively measure inflammation of the surface of the eyeball. The test measures MMP-9, which is specific for inflammation of the ocular surface and is recognized with a determinant factor in the chronic inflammation that occurs in dry eye. If dry eye treatment is effective, this test should be negative in subsequent tests;

- Impression cytology sampling. Through this study, a pathologist can assess the presence of inflammatory cells on the ocular surface (conjunctiva). If dry eye treatment is effective, it should negate the presence of these cells in the conjunctiva in subsequent measurements;

- Ocular surface staining with sodium fluorescein (AK-Fluor® Akorn, Lake Forest, IL, USA) and lissamine green (Rose Stone Enterprises, Alta Loma, CA, USA) to evaluate the surface of the cornea and conjunctiva of the SICCA (Sjogren Clinical Collaboration Alliance) OCT (Ocular Staining Score) scale was used. These stains are used by the ophthalmology community to evaluate the severity and response to treatment of dry eye syndrome;

- Schirmer test (Eagle Vision, Inc., Memphis TN, USA). This test is used by the ophthalmology community to evaluate the severity and response to treatment of dry eye syndrome;

- Intraocular pressure measurement (mmHg) (Icare® TA01i tonometer, Belleville, MI, USA). This test is part of the standard examination that every ophthalmologist performs on their patients;

- Evaluation of the fundus with indirect ophthalmoscopy (Killer Vantage Plus LED, Malvern, PA, USA). This test is part of the standard examination that every ophthalmologist performs on their patients.

To ensure the objectivity of these measurements, all were carried out at the same morning time (09:00 am to 11:00 am), in the same room, with silence, humidity control (40-50%) and the same controlled temperature (23-24°C), without use of air conditioning during the review time and by the same researcher. Patients' adherence to the use of study medications (including placebo) was assessed based on a written diary.

$$AD = (RA)100/IA$$

where AD is adherence, RA is the number of records of use of study medications and IA is the number of their administrations. An adherence value of <90% was considered a failure in the adherence of these subjects to the treatment indicated in their study group. If this was the case, these subjects would be excluded from the study. Figure 2 demonstrates the design of the efficacy study. During the study, all subjects attended a baseline visit and were assigned to 1 of 2 groups; all had a minimum follow-up period of 4 weeks (28 days).

**Results.**

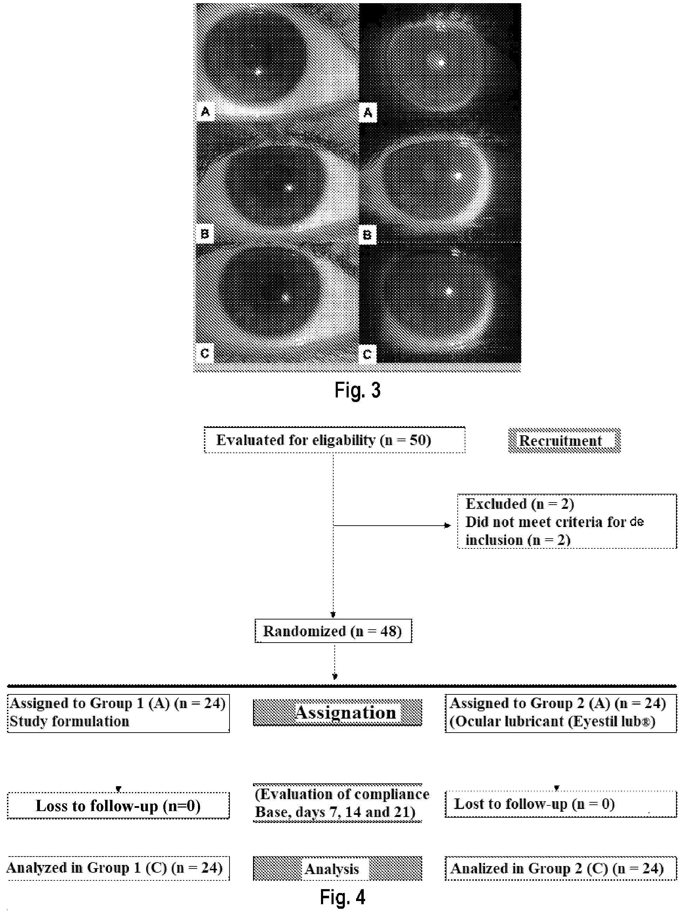

**1. Tolerability and safety study.** During the entire follow-up time, no adverse events were recorded. The formulation was well tolerated by all study subjects (result of the ocular tolerability questionnaire). The study proved that the formulation is tolerable and safe for use in humans. Figure 3 has representative images of the safety study; The images show the same eye of the same patient in its basal shot (A) with (blue light) and without (white light), and in the week 1 (B) and week 3 (C) shot, the results demonstrate that there were no changes in ocular surface staining after the use of the study formulation.

**2. Efficacy study.** The study included 48 eyes of 48 patients with mild to moderate dry eye disease (DED) who met all inclusion criteria and none of the exclusion criteria. The data of 48 subjects were analyzed (24 for group A and 24 for group C), thus all completed the minimum follow-up time with an adherence of all subjects (100%) of >90% to the use of the treatment. Regarding this efficacy study, there was a statistically significant difference between baseline and month 1 (day 28) in OSDI (ocular surface disease index) test results and breakthrough time. non-invasive tear test (NIF-BUT) in both treatment groups (group A and group C). However, only the study group (group A) showed statistically significant differences between baseline (baseline) and month 1 (day 28) with respect to the average non-invasive tear breakup time (NIAvg-BUT) score and Schirmer test between baseline intake and day 28 of follow-up. Regarding the objective measurement of ocular inflammation (measurement of MMP-9 with the Quidel InflammaDry® test), the study group exhibited a statistically significant decrease in the number of eyes with a positive test (100% at baseline intake vs. 33.4% on day 28 of follow-up), while in the group using ocular lubricant (Eyestil lub®) this was not the case (100% baseline intake vs. 83% on day 28 of follow-up). This data is of great relevance, because the objective and conclusive measurement of inflammation of the ocular surface demonstrates the anti-inflammatory activity of our formulation for ophthalmic topical use that contains Helenalin, 11-$\alpha$, 13-dihydrohelenalin and flavonoids from Arnica Montana extract in combination with sodium hyaluronate. Likewise, the conjunctival impression cytology test demonstrated significant changes in the study group of the formulation (group A) after the corresponding pathology analysis, while the group using ocular lubricant (group C) did not exhibit significant changes. These last two results confirm that the addition of Helenalin, 11-$\alpha$, 13-dihydrohelenalina and flavonoids from the Arnica Montana extract to sodium hyaluronate provides anti-inflammatory activity that sodium hyaluronate alone does not possess (statistically significant results from group A versus the non-significant results from group C).

Finally, ocular surface staining tests exhibited a statistically significant difference in the study formulation group (group A), but not in the control group (group C). Figure 4 graphically illustrates the efficacy study, the diagram demonstrates the subjects enrolled in the efficacy study who were admitted, randomized and analyzed in both groups (group A = study formulation and group C = control with ocular lubricant (Eyestil lub®)).

Table 1 represents the demographic and baseline data of the efficacy study (with standard deviation). All patients in both groups presented diagnostic criteria for mild to moderate dry eye syndrome and all reacted positive in the MMP-9 test.

**Table 1.**

| | A (Formulation of the study) | C (Control/Eye Lubricant) |
|---|---|---|
| Age | 53.1 ± 9.82 | 52.5 ± 13.15 |

(continued)

|  | A (Formulation of the study) | C (Control/Eye Lubricant) |
|---|---|---|
| Gender |  |  |
| Male (n) | 13 | 12 |
| Female (n) | 11 | 12 |
| Hypertension (n) | 1 | 0 |
| Ocular findings |  |  |
| Pseudophakic (n) | 5 | 3 |
| Baseline BCVA |  |  |
| (ETDRS letters) | 82.5 ± 4.2 | 82.2 ± 5.1 |
| OSDI (score) | 20.82 ± 6.55 | 21.24 ± 5.36 |
| NIF-BUT (s) | 7.65 ± 4.52 | 8.11 ± 6.15 |
| NIAvg-BUT (s) | 8.21 ± 3.22 | 8.23 ± 3.44 |
| Schirmer test (mm) | 10.84 ± 47 | 11.32 ± 33 |
| MMP-9 Test Positivity | 100% | 100% |

Table 2 represents the data from the efficacy study. The subjects enrolled in the efficacy study were admitted, randomized and analyzed in both groups (group A = study formulation and group C = control with ocular lubricant (Eyestil lub®)). The improvement in OSDI and NIF-BUT was statistically significant in both study groups (both groups used lubricant (Sodium hyaluronate).

However, only the subjects in group A exhibited a statistically significant improvement in the NIAvg-BUT, the Schirmer test and, especially, in the measurement of MMP-9, this last test being specific for the measurement of inflammation on the surface of the eyeball, demonstrating the unique and unexpected anti-inflammatory activity of the formulation.

Baseline = baseline intake, Month 1 = follow-up month 1, *Statistically significant difference.

**Table 2.**

| | Group A | | Group C | |
|---|---|---|---|---|
| Variable/Visit | Baseline | Month 1 | Baseline | Month 1 |
| OSDI (score) | 22.31 ± 3.32 | 10.58 ± 4.21* | 21.74 ± 15 | 11.23 ± 8.95* |
| NIF-BUT (s) | 8.39 ± 5.18 | 14.53 ± 4.53* | 8.43 ± 4.82 | 13.83 ± 5.69* |
| NIAvg-BUT (s) | 10.46 ± 4.19 | 14.24 ± 3.66* | 9.85 ± 4.13 | 11.73 ± 5.82 |
| Schirmer test (mm) | 15.83 ± 5.4 | 20.05 ± 4.37* | 16.11 ± 5.1 | 17.83 ± 6.28 |
| Elevated MMP-9 | 24/24 (100% ) | 6/24 (25%)* | 24/24 (100%) | 21/24 (87.5%) |

Table 3 represents the quantitative analysis of ocular surface stains. In relation to ocular surface staining, group A exhibited a statistically significant difference, and this is particularly relevant since this marker is specific to chronic damage to the ocular surface due to dry eye. Group C did not statistically significant improvement in corneal staining. Baseline = baseline intake, Month 1 = follow-up month 1, *Statistically significant difference.

**Table 3.**

| | Group A | | Group C | |
|---|---|---|---|---|
| Grade/Visit | Baseline | Month 1* | Baseline | Month 1 |
| 0 | 2 | 18 | 1 | 9 |
| I | 10 | 6 | 11 | 11 |
| II | 9 | 0 | 7 | 5 |
| III | 3 | 0 | 7 | 1 |
| IV | 0 | 0 | 0 | 0 |
| V | 0 | 0 | 0 | 0 |

Table 4 shows the quantitative analysis of the results of the ocular surface impression cytology test, where the study group showed statistically significant improvement between the baseline measurement and that on day 28. Likewise, the study group showed statistically significant improvement vs. the results obtained in the control group. Finally, the control group did not exhibit any improvement between the baseline intake and the intake on day 28.

| | Study Group (A) | | Control Group (C) | |
|---|---|---|---|---|
| Subject Number | Basal | Day 28 | Basal | Day 28 |
| 1 | 1 | 0 | 1 | 1 |
| 2 | 2 | 0 | 1 | 1 |
| 3 | 1 | 0 | 2 | 2 |
| 4 | 1 | 0 | 1 | 2 |
| 5 | 2 | 0 | 2 | 2 |
| 6 | 3 | 0 | 1 | 1 |
| 7 | 2 | 0 | 2 | 2 |
| 8 | 2 | 0 | | |

**\* Statistically significant difference**

**Table 4**

| Group | Analysis | Test | p Value |
|---|---|---|---|
| Study group 1 (n = 8) | Baseline vs Month 1 | Wilcoxon signed rank test of paired groups | 0.0078* |
| Control Group 2 (n = 7) | Baseline vs Month 1 | Wilcoxon signed rank test of paired groups | >0.9999 |
| Study Group 1 (n = 8) vs Control Group 2 (n = 7) | Baseline vs Baseline | Mann-Whitney Test | 0.5051 |
| Group 1 (n = 8) vs Control Group 2 (n = 7) | Month 1 vs Month 1 | Mann-Whitney Test | 0.002* |

Therefore, our data demonstrate that this formulation for ophthalmic topical use containing Helenalin, 11-$\alpha$, 13-dihydrohelenalin and flavonoids from Arnica Montana extract in combination with sodium hyaluronate has anti-inflammatory activity and properties (statistically significant results in measurement tests of MMP-9 from the Quidel InflammaDry® test and conjunctival impression cytology tests), increases the quality of the tear film, statistically significant results of non-invasive measurements of tear breakup time (NI F-BUT), average tear breakup time (NIAvg-BUT) and Schirmer test, improves the condition of the tissues of the surface of the eyeball (conjunctiva staining tests with lissamine green and cornea with sodium fluorescein), decreases the evaporation of tear (statistically significant results of non-invasive measurements of tear breakup time (NIF-BUT), average tear breakup time (NIAvg-BUT) and improves symptoms associated with dry eye syndrome and monitor use condition (statistically significant results of the OSDI test).

In summary, our invention of a formulation for topical ophthalmic use that contains helenalin, 11-$\alpha$, 13-dihydrohelenalin and flavonoids from Arnica Montana extract in combination with sodium hyaluronate for the treatment of dry eye syndrome in humans is tolerable, safe and effective in reducing the symptoms associated with dry eye syndrome, has anti-inflammatory effects that inhibit inflammation of the surface of the eyeball and increases the quality of the tear film in people with dry eye syndrome and fatigue from using monitors (computer vision syndrome - or visual strain). Additionally, in the results obtained, the capacity of the formulation was verified both in the lower and upper range of values of the Arnica Montana extract which extended from 100 to 500 mg per 1 mL. For the upper value, which is 500 mg per 1 mL and which was used in a range of 500 to 1000 mg per 1 mL of the formulation, no response comparable to the values obtained with the initial formulation was found. For the lower value which is 100 mg per 1 mL in the original formulation, lower values were tested within a range of 2 to 100 mg per 1 mL of the formulation and in this case comparable responses to the initial formulation were found. In this way, there is evidence that Arnica Montana extract can be in the range of 2 to 100 mg per 1 ml of the formulation without affecting the properties for use in dry eye syndrome. In this way it can be established that the distribution of the behavior of the values of the Arnica Montana extract is biased towards the lower values of the range with values ranging from 2 to 500 mg per 1 mL of the formulation that includes its components.

**Conclusions.**

Based on the results obtained in the clinical study designed to evaluate the tolerability, safety and efficacy profile, the generation of proof of concept for the topical administration of the ophthalmic topical use formulation containing helenalin, 11-$\alpha$, 13-dihydrohelenalin and flavonoids from Arnica Montana extract in combination with sodium hyaluronate for treatment of dry eye syndrome in humans, as well as proof of the amelioration effect on the quality of the tear film (measurement of NIF-BUT, NIAvg-BUT, Schirmer test), modulation of the inflammatory process (measurement of MMP-9, staining of eyeball surface of the cornea and conjunctiva and measurement of meibomian gland inflammation) and amelioration of symptoms associated with dry eye syndrome (OSDI) was enabled.

These favorable, unexpected and not previously described results from a clinical study demonstrate the anti-inflammatory and antioxidant properties, as well as an improvement in the quality of the tear film, modulation the inflammatory process and amelioration of the symptoms associated with dry eye syndrome of the described formulation, which comprises:

a) A component with anti-inflammatory and antioxidant properties and a lubricant containing helenalin, 11-$\alpha$, 13-dihydrohelenalin and flavonoids from *Arnica Montana* extract in combination with sodium hyaluronate on a 1 ml basis that includes:

i. Arnica Montana Extract: 100-500 mg;

ii. Sodium hyaluronate: 1 -4 mg

This formulation has an adequate safety profile and is well tolerated, as well as a clinically significant efficacy profile and ameliorates the symptoms associated with inflammation of the ocular surface, as well as improving the signs associated with it, such as de-epithelialization of cornea and conjunctiva, tear rupture time and inflammation of the meibomian glands (measured with the Schwind Sirius® equipment), as well as having anti-inflammatory efficacy reducing the presence of MMP-9 on the surface of the eyeball.

**Impression cytology of the ocular surface: morphological changes in the conjunctiva.**

**Background.**

Impression cytology (IC) is a technique that allows the recovery of the outermost layer of cells from the ocular surface through the use of various types of filters. It is a minimally invasive method to evaluate the morphology of human conjunctival epithelial cells in the diagnosis of dry eye disease, a common and distressing disorder associated with aging, contact lens use, autoimmune disorders, and refractive surgery (LASIK: laser-assisted in situ keratomileusis). Recently, it has been used in dry eye disease.

IC has traditionally been used to identify morphological changes (for example, squamous metaplasia) in ocular surface diseases, mainly dry eye disease. However, later IC studies evaluated other ocular surface diseases and observed substantial loss of conjunctival goblet cells and changes in epithelial cell size. Several groups have used IC to evaluate the effects of dry eye disease therapies on the ocular surface, including the effects of preservatives in ophthalmic medications and autologous serum.

**Methods.**

All subjects (study group and control group) underwent IC in the left eye. The IC procedure included membrane impression cytology.

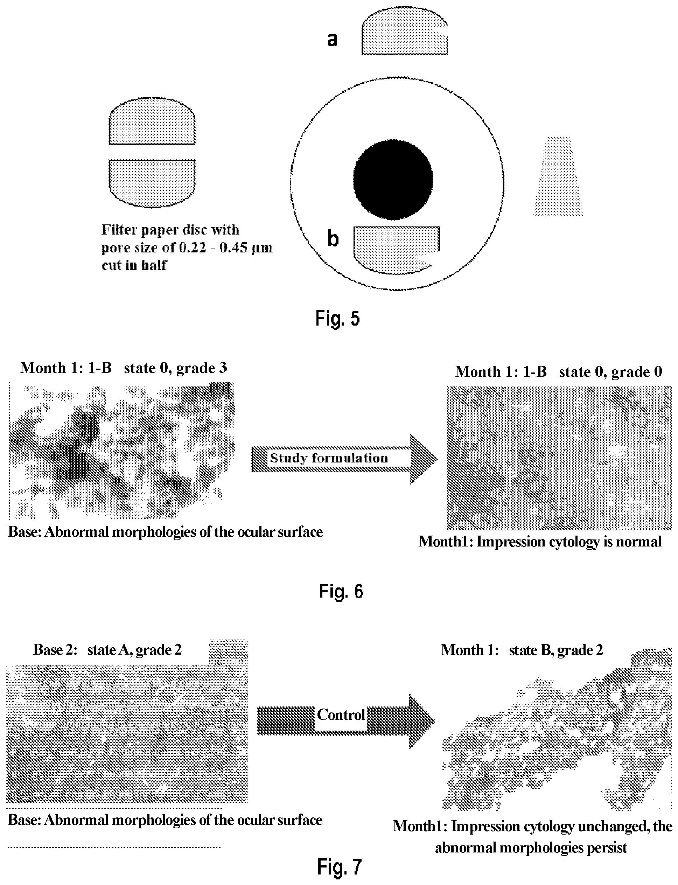

Millipore, Papanicolaou stain and Nelson grading. The technique used is illustrated in Figure 5. This figure demonstrates a filter paper disc cut into halves (left) and the application of the resulting D-shaped segments (trimmed for orientation **a and b**) to the bulbar conjunctiva and cornea using the head of the Goldmann tonometer to apply uniform pressure on the filter paper (right). The content of 11-$\alpha$, 13-dihydrohelenalin was at a concentration ranging from 0.004% to 5% based on 1 ml for use in dry eye syndrome.

**Results.**

Eight of the 24 subjects (33.3%) in the study group and 7 of the 24 subjects (29.1%) in the control group exhibited abnormalities in ocular surface morphology. All subjects in the study group presented with normal impression cytology after 1 month of treatment with the study drops. However, none of the eyes in the control group exhibited improvement in impression cytology after 1 month of ocular lubricant treatment.

In the study group, a statistically significant improvement in ocular surface morphology was observed by impression cytology 1 month after treatment with AcuSense drops®. Such improvement was not observed in the control group.

Table 5 represents Cytology impression of the ocular surface. Study group vs Control group. After 1 month of treatment, all eyes with ocular surface morphological abnormalities exhibited improvement in the study group. None of the eyes in the control group exhibited improvement.

**Table 5**

| Subject number | Study Group 1 | | Study Group 2 | |
|---|---|---|---|---|
| | Basal | Month 1 | Basal | Month 1 |
| 1 | 1 | 0 | 1 | 1 |
| 2 | 2 | 0 | 1 | 1 |
| 3 | 1 | 0 | 2 | 2 |
| 4 | 1 | 0 | 1 | 2 |
| 5 | 2 | 0 | 2 | 2 |
| 6 | 3 | 0 | 1 | 1 |
| 7 | 2 | 0 | 2 | 2 |
| 8 | 2 | 0 | | |

Table 6 presents the statistical analysis of the study. There was a significant statistical difference in the impression cytology of the study group (baseline vs month 1) and study group vs control group. The control group did not exhibit a significant statistical difference.

**Table 6.**

| Group | Analysis | Test | p Value |
|---|---|---|---|
| Study group 1 (n = 8) | Baseline vs Month 1 | Wilcoxon signed rank test of paired groups | 0.0078* |
| Control Group 2 (n = 7) | Baseline vs Month 1 | Wilcoxon signed rank test of paired groups | >0.9999 |
| Study Group 1(n = 8) vs Control Group (n = 7) | Baseline vs Baseline | Mann-Whitney Test | 0.5051 |
| Group 1 (n = 8) vs Control Group 2 (n = 7) | Month 1 vs Month 1 | Mann-Whitney Test | 0.0002* |
| ***Statistically significant** | | | |

After 1 month of treatment with AcuSense drops® there was an improvement in the morphology of the ocular surface. Patients with morphological abnormalities of the conjunctival mucus and epithelium exhibited normal impression cytology at the end of follow-up.

**Study group:** Eight of 24 (33.3%) eyes in the study group presented with morphologically abnormal ocular surfaces at baseline. All of them exhibited normal impression cytology after 1 month of treatment with AcuSense drops®. Figure 6 illustrates Millipore membrane impression cytology, Nelson grading, Papanicolaou stain.

**1A**: BASELINE, Stage 0, Grade 3. Abundant cellularity, goblet cells of 80-200 cells per $mm^2$, moderately frequent intercellular separations with modified cytoplasmic-nucleus ratio in favor of the cytoplasmic. Mild inflammation, slightly increased apoptosis.

**1-B**: MONTH 1, Stage 0, Grade 0. Abundant cellularity is observed, goblet cells of 400 or more cells per $mm^2$, cohesive with a normal cytoplasmic nucleus ratio. There are no inflammatory cells or apparent apoptosis.

None of the eyes with morphological abnormalities on the ocular surface in the control group exhibited improvement after 1 month of treatment with ocular lubricant.

**Control group:** 7 of 24 (29.1%) eyes in the study presented with morphologically abnormal ocular surfaces at baseline. None of them exhibited improvement in impression cytology after 1 month of ocular lubricant treatment. Figure 7 illustrates Millipore membrane impression cytology, Nelson grading, Papanicolaou stain.

**2-A**: BASELINE, Stage 0, Grade 1. Abundant cellularity, goblet cells of 300-400 cells per $mm^2$, cohesive or discrete intercellular separations with normal nucleocytoplasmic ratio. Minimal inflammation, minimal apoptosis.

2-B: Month 1, Stage 0, Grade 2. Abundant cellularity, goblet cells of 200-300 cells per mm$^2$, frequent slight intercellular separations with modified cytoplasmic-nucleus ratio in favor of the cytoplasmic. Mild inflammation, slightly increased apoptosis.

**Conclusion.**

The use of the study drops (*Arnica Montana* Extract + hyaluronic acid) improved IC to normal. Regular ocular lubricant did not improve morphological abnormalities in IC. These results should be considered objective evidence of the therapeutic effect of the bioactivities present in the extract of *Arnica Montana* (Helenalin and 11-$\alpha$, 13-dihydrohelenalin) on the ocular surface in subjects with dry eye disease.

*Use of a validated formulation to improve the quality of the tear film, modulate the inflammatory process and the symptoms associated with dry eye syndrome of the formulation based on helenalin, 11-$\alpha$, 13-dihydro-helenalin and flavonoids from Arnica Montana extract in combination with sodium hyaluronate.*

Regarding the verification presented in the previous study, the formulation that is claimed as an invention is the following:

An ophthalmic formulation for use in treating symptoms associated with dry eye syndrome that contains on a 1 ml basis:

    a) Arnica Montana Extract: 100-500 mg;
    b) Sodium hyaluronate: 1-4 mg.

Additionally, the formulation was validated for use in:

- Optimization of the presence of bio-active elements present in *Arnica Montana,* through extraction processes using ethanol, ethyl ether, acetone, chloroform and petroleum ether for use in dry eye syndrome;

- Where the content of 11-$\alpha$, 13-dihydrohelenalin is in a concentration ranging from 0.004% to 5% based on 1 ml for use in dry eye syndrome;

- In the blockade of the nuclear transcriptional factor NF-kB and the inhibition of the expression of inflammatory interleukin and matrix metalloproteinases on the surface of the eyeball in humans with dry eye;

- The decrease in morphological abnormalities of the surface of the ocular globe associated with the inflammatory process present in dry eye syndrome in humans;

- Decreased evaporation of the tear film in humans with dry eye syndrome;

- The decrease in conjunctival de-epithelialization in humans with dry eye syndrome;

- Increasing tear film stability in humans with dry eye syndrome;

- Modulation of the inflammatory process associated with dry eye syndrome in humans;

- The reduction of symptoms generated by the inflammatory process associated with dry eye syndrome in humans;

- Where the formulation can be sterilized by heat-steam method, ultrafiltration and gamma rays for use in dry eye syndrome;

- Where the formulation may contain preservatives such as benzalkonium chloride, benzyl alcohol or be preservative free for use in dry eye syndrome;

- Where the formulation *may be manufactured as an herbal medicine for use in dry eye syndrome;*

- Where the formulation may *be manufactured as a medical device for use in dry eye syndrome;*

- Where the formulation may be contained in bottles specially designed for preservative-free topical ophthalmic products for use in dry eye syndrome.

The formulation additionally has anti-inflammatory, antioxidant and lubricating properties within ophthalmological applications and in the treatment of dry eye syndrome. The above description of the disclosed definitions is provided to enable any person skilled in the art to make or use the present invention. Various modifications to these definitions and/or implementations will be readily apparent to those skilled in the art, and the generic principles defined herein may be applied to other embodiments without departing from the spirit or scope of the invention. Thus, the present invention is not intended to be limited to the embodiments disclosed herein, but should be granted the broadest scope consistent with the following claims and the principles and novel features described herein.

**Claims**

1.  A topical ophthalmic formulation containing helenalin, 11-$\alpha$, 13- dihydrohelenalin and flavonoids from *Arnica Montana* extract in combination with sodium hyaluronate for the treatment of dry eye syndrome in humans **characterized by** containing per 1 mL:

    a. Arnica Montana Extract: 100-500 mg,
    b. Sodium hyaluronate: 1-4 mg.

2.  The topical ophthalmic formulation according to claim 1, where the bioactivities of Arnica Montana are obtained through extraction processes using ethanol, ethyl ether, acetone, chloroform and petroleum ether for use in dry eye syndrome.

3.  The topical ophthalmic formulation according to claim 1, wherein the lower range of the Arnica Montana extract ranges from 2 to 100 mg per 1 mL for use in dry eye syndrome.

4.  The topical ophthalmic formulation according to claim 1, wherein the content of 11-$\alpha$, 13-dihydrohelenalin is at a concentration ranging from 0.004% to 5% based on 1 mL for use in dry eye syndrome.

5.  The topical ophthalmic formulation according to claim 1, **characterized in that** it has anti-inflammatory, antioxidant and lubricating properties.

6.  The topical ophthalmic formulation according to claim 1, for use in blocking the nuclear transcriptional factor NFkB and inhibiting the expression of inflammatory interleukins and matrix metalloproteinases on the surface of the eyeball in humans with dry eye.

7.  The topical ophthalmic formulation according to claim 1, for use in reducing morphological abnormalities of the surface of the eyeball associated with the inflammatory process present in dry eye syndrome in humans.

8.  The topical ophthalmic formulation according to claim 1, for use in decreasing the evaporation of the tear film of humans with dry eye syndrome.

9.  The topical ophthalmic formulation according to claim 1, for use in reducing conjunctival de-epithelialization in humans with dry eye syndrome.

10. The topical ophthalmic formulation according to claim 1, for use in increasing the stability of the tear film in humans with dry eye syndrome.

11. The topical ophthalmic formulation according to claim 1, for use in modulating the inflammatory process associated with dry eye syndrome in humans.

12. The topical ophthalmic formulation according to claim 1, for use in reducing the symptoms generated by the inflammatory process associated with dry eye syndrome in humans.

13. The topical ophthalmic formulation according to claim 1, which can be sterilized by a heat-steam method, ultrafiltration and gamma rays for use in dry eye syndrome.

14. The topical ophthalmic formulation according to claim 1, which may contain preservatives such as benzalkonium chloride, benzyl alcohol or be preservative-free for use in dry eye syndrome.

15. The topical ophthalmic formulation according to claim 1, which may be contained in bottles specially designed for preservative-free topical ophthalmic products for use in dry eye syndrome.

Fig. 1

Fig. 2

Fig. 3

Fig. 4

a

Filter paper disc with
pore size of 0.22 - 0.45 µm
cut in half

b

Fig. 5

**Month 1: 1-B   state 0, grade 3**

Study formulation

**Month 1: 1-B   state 0, grade 0**

Base: Abnormal morphologies of the ocular surface

Month1: Impression cytology is normal

Fig. 6

**Base 2:   state A, grade 2**

Control

**Month 1:   state B, grade 2**

Base: Abnormal morphologies of the ocular surface

Month1: Impression cytology unchanged, the
abnormal morphologies persist

Fig. 7

**EP 4 631 513 A1**

| INTERNATIONAL SEARCH REPORT | International application No. |
|---|---|
| | PCT/MX2022/050122 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, MEDLINE, NPL, EMBASE, BIOSIS

**C. DOCUMENTS CONSIDERED TO BE RELEVANT**

| Category* | Citation of document, with indication, where appropriate, of the relevant passages | Relevant to claim No. |
|---|---|---|
| X | BUZZONETTI L. et al. Effectiveness of hyaluronic acid and arnica extract ophthalmic solution in reducing dry eye symptoms in pediatric population. European Journal Of Ophthalmology, 02/10/2022, Vol. 33, N° 2, pages 1011-1017, the whole document | 1 |
| X | Arnistil. Doc Generici S.r.l., 28/06/2022 [on line][retrieved on 08/06/2023]. Retrieved of <URL: https://www.docgenerici.it/doc-ofta-oftalmologia/i-prodotti-doc-ofta/sostituti-lacrimali/arnistil/>, the whole document | 1 |
| X | MX 2017001879 A (CENTRO DE RETINA MÉDICA Y QUIRÚRGICA S) 09/08/2018, the whole document | 1 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 09/06/2023 | **(14/06/2023)** |

| Name and mailing address of the ISA/ | Authorized officer |
|---|---|
| | M. Cumbreño Galindo |
| OFICINA ESPAÑOLA DE PATENTES Y MARCAS Paseo de la Castellana, 75 - 28071 Madrid (España) Facsimile No.: 91 349 53 04 | Telephone No. 91 3496880 |

Form PCT/ISA/210 (second sheet) (July 2022)

## INTERNATIONAL SEARCH REPORT

| International application No. |
| --- |
|  |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.:
because they relate to parts of the international application that do not comply with the prescribed requirements to such an extent that no meaningful international search can be carried out, specifically:

The subject matter of claims 2-15 fails to meet the requirements of clarity and conciseness (PCT Article 6). In addition, claims 2-4 and 13-15 are not fully supported by the description and thereby contravene PCT Article 6.

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.  Consequently, this international search report is restricted to the invention first mentioned in the claims;  it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## INTERNATIONAL SEARCH REPORT

Information on patent family members

| International application No. |
| --- |
| PCT/MX2022/050122 |

| Patent document cited in the search report | Publication date | Patent family member(s) | Publication date |
| --- | --- | --- | --- |
| MX2017001879 A | 09.08.2018 | NONE | |

Form PCT/ISA/210 (patent family annex) (July 2022)

## INTERNATIONAL SEARCH REPORT

| International application No. |
| --- |
| PCT/MX2022/050122 |

CLASSIFICATION OF SUBJECT MATTER

*A61K36/28* (2006.01)
*A61K31/728* (2006.01)
*A61P27/02* (2006.01)

Form PCT/ISA/210 (extra sheet) (July 2022)

**EP 4 631 513 A1**

REFERENCES CITED IN THE DESCRIPTION

**Patent documents cited in the description**

- US 10231990 B
- CN 107929198
- MX 390524